# EUROPÄISCHE PATENTANMELDUNG

(11) **EP 1 312 311 A1**
(43) Veröffentlichungstag der Anmeldung: **21.05.2003**
(21) Anmeldenummer: 02025807.5
(22) Anmeldetag: 18.11.2002
(51) Int. Cl.: A61B 10/00, A01K 1/01

(54) **Verfahren und Einrichtung zur Bestimmung des Gesundheitsstatus von Tieren**

(30) Priorität: 16.11.2001 DE 10156453
(71) Anmelder: Westfalia Landtechnik GmbH, 59302 Oelde (DE)
(72) Erfinder: Redetzky, Ralf, 30559 Hannover (DE)
(74) Vertreter: Neumann, Ditmar

(57) **Zusammenfassung**

Zur Bestimmung eines Gesundheitsstatus eines Tieres wird ein Verfahren und eine Vorrichtung vorgeschlagen. Wenigstens ein Teil eines Exkrements des Tieres wird aufgefangen. Aus dem aufgefangenen Exkrement wird wenigstens eine physikalische Kenngröße ermittelt. Bei dieser Kenngröße kann es sich um die Temperatur, elektrische Leitfähigkeit oder Viskosität des Exkrements handeln. Anhand der physikalischen Kenngröße wird überprüft, ob die physikalische Kenngröße innerhalb eines vorgegebenen Toleranzfeldes liegt, so dass Aufschluß darüber gewonnen werden kann, welchen Gesundheitsstatus ein Tier hat.

## Beschreibung

Der Gegenstand der Erfindung bezieht sich auf ein Verfahren sowie auf eine Einrichtung zur Bestimmung des Gesundheitsstatus von Tieren.

In der Milchtierwirtschaft unterliegen die Tiere, die Milch abgeben, einer strengen gesundheitlichen Kontrolle, da die Rohmilch, die die Tiere abgeben, gegebenenfalls nach weiterer Verarbeitung als Lebensmittel für den menschlichen Verzehr verwendet wird. So dürfen Kühe, Büffel, Schafe, Ziegen und Stuten, von denen Milch als Lebensmittel gewonnen wird, keine erkennbaren Anzeichen einer Störung des Gesundheitszustandes aufweisen. Hierbei ist nicht nur der Gesundheitszustand des Euters sondern der Gesundheitszustand des gesamten Tieres von Bedeutung.

Es ist bekannt, dass über die Analyse von Milch, die ein Tier abgibt, bei entsprechender Auswahl geeigneter Parameter Aussagen zum Eutergesundheitszustand des Tieres getroffen werden können. Aussagen zum Allgemeingesundheitszustand von Tieres sind über die Milch jedoch nur eingeschränkt möglich. So können beispielsweise bestimmte Stoffwechselstörungen und Fütterungsfehler wie Ketosen und bedingt auch Acidosen über die Messung von Fett, Eiweiss und Ketonkörpern ermittelt werden. Andere Erkrankungen lassen sich über die Milch nicht oder nur unzureichend erfassen. Die Erfassung erfolgt dann mit einem nicht unerheblichen labortechnischen Aufwand.

Der Gesetzgeber verlangt in der Anlage 1 der Milchverordnung jedoch, das Tiere, von denen Milch als Lebensmittel gewonnen wird, bestimmte Anforderungen erfüllen müssen. Unter anderem dürfen sie keine Anzeichen von ansteckenden, durch die Milch auf Menschen übertragbaren Krankheiten aufweisen. Ferner dürfen sie keine erkennbaren Anzeichen einer Störung des allgemeinen Gesundheitszustandes aufweisen und nicht an Magen-Darm-Krankheiten leiden. Einige Infektionen sind auch von humanmedizinischer Bedeutung, beispielsweise Salmonellose, Para-TBC beim Rind, John'sche Krankheit - Morbus Crohn. Weitere Erkrankungen können mit Störungen des Allgemeinbefindens einhergehen. Ein Symptom für solche Störungen ist der Anstieg der Körpertemperatur (Fieber).

Der vorliegenden Erfindung liegt daher die Zielsetzung zugrunde, ein Verfahren und eine Einrichtung anzugeben, mit deren Hilfe ein Beitrag zur Bestimmung des allgemeinen Gesundheitszustandes von Tieren, insbesondere von Rindern, geleistet werden kann.

Diese Zielsetzung wird erfindungsgemäß durch ein Verfahren mit den Merkmalen des Anspruchs 1 bzw. durch eine Einrichtung mit den Merkmalen des Anspruchs 9 erzielt. Vorteilhafte Weiterbildungen und Ausgestaltungen des Verfahrens bzw. der Einrichtung sind Gegenstand der jeweiligen abhängigen Patentansprüche.

Der vorliegenden Erfindung liegt die prinzipielle Überlegung zugrunde, dass durch eine Analyse wenigstens eines Teils eines Exkrements des Tieres Schlussfolgerungen über den Gesundheitszustand eines Tieres gezogen werden können. Im Konkreten wird bei dem erfindungsgemäßen Verfahren vorgeschlagen, dass wenigstens ein Teil eines Exkrements des Tieres aufgefangen wird. Der Auffangvorgang erfolgt vorzugsweise unmittelbar nach dem Ausscheidevorgang des Exkrements. Das aufgefangene Exkrement wird hinsichtlich wenigstens einer vorzugsweise physikalischen Kenngröße analysiert. Die so gewonnenen physikalischen Kenngrößen werden vorzugsweise gespeichert und dahingehend überprüft, ob wenigstens die eine physikalische Kenngröße innerhalb eines vorgegebenen Toleranzfeldes liegt. Ist dies der Fall, so kann ein positiver Gesundheitsbefund dem Tier attestiert werden. Führt die Überprüfung ob wenigstens eine physikalische Kenngröße innerhalb eines vorgegebenen Toleranzfeldes liegt, dazu, dass diese physikalische Kenngröße außerhalb des Toleranzfeldes liegt, so kann von einer pathologischen Veränderung des Gesundheitszustandes des Tieres ausgegangen werden. Die Veränderung des Gesundheitszustandes kann bei Verwendung der Milch eines solchen Tieres möglicherweise zu einem Verstoß gegen bestehende Verordnungen führen. Gegebenenfalls kann das Tier nach diesem Befund weiteren Untersuchungen unterzogen werden. Durch geeignete Maßnahmen kann das Tier aus dem Herdenverband selektiert werden.

Die Erfindung kann in manuellen Melkständen, halb- oder auch vollautomatischen Einrichtungen zum Melken, Reinigen, Füttern oder dergleichen verwendet werden. Der Einsatz bei automatischen Systemen ist insbesondere vorteilhaft. Ebenso kann die Erfindung auch in Fressstationen, z.B. im Stallbereich zur Abgabe von Grundfutter und/oder Kraftfutter, eingesetzt werden.

Laut Milchgüteverordnung der Europäischen Union darf die Milch von Tieren, die von Durchfallerkrankungen betroffen sind, nicht verwendet werden. Die erfindungsgemäße Einrichtung und das erfindungsgemäße Verfahren sind sehr vorteilhaft, da damit erkrankte Tiere erkannt werden können. Insbesondere, aber nicht nur, in automatisch arbeitenden Melkanlagen ist das sehr vorteilhaft.

In einer bevorzugten Weiterbildung wird wenigstens ein Teil des Kotes untersucht. Es ist aber auch möglich, dass nur der ausgeschiedene Harn untersucht wird. Auch die Untersuchung von Harn und Kot und weiterer Exkremente ist möglich.

Um eine relativ hohe diagnostische Sicherheit zu erhalten, ist es von Bedeutung, dass die Exkremente eines Tieres für die Ermittlung und Speicherung wenigstens einer physikalischen Kenngröße des Exkrements nicht mit den Exkrementen eines anderen Tieres vermischt werden. Es wird daher vorgeschlagen, dass vor dem Auffangen des Exkrements eines Tieres die Auffangeinheit, die zum Auffangen wenigstens eines Teils eines Exkrements des Tieres vorgesehen ist, gereinigt wird. Die Reinigung kann über eine mechanische Separation oder ein Abstreifen oder dergleichen erfolgen.

Insbesondere wird vorgeschlagen, dass die Auffangeinheit mit einem Fluidmittel gereinigt wird. Als Fluid kommt vorzugsweise eine Flüssigkeit in Betracht. Es ist auch möglich, dass z.B. nach einer mechanischen Reinigung eine zusätzliche Reinigung mit Wasser und/oder Druckluft erfolgt. Eine Reinigung mit einer Flüssigkeit kann zusätzlich zu einer mechanischen Reinigung oder an Stelle einer mechanischen Reinigung erfolgen.

Der Zeitpunkt, für den Reinigungsbeginn kann durch andere Einrichtungen bestimmt werden. Hierbei kann es sich beispielsweise um Signalgeber handeln, die Bauteile von Melkboxen oder Melkplätzen sind. Insbesondere kann es sich hierbei um eine Milchflusserkennungseinheit, Melkzeugabnahmeeinheit, eine Tor- oder Tiersteuerung oder eine Tiererkennung im Ausgangsbereich eines Melkplatzes handeln. Es ist z.B. möglich, einen Signalgeber einer vorderen Tür zu verwenden.

Nach noch einer weiteren vorteilhaften Weiterbildung des Verfahrens wird vorgeschlagen, dass der Reinigungsvorgang wiederholt wird, wenn eine Überprüfung ergibt, dass der vorhergehende Reinigungsvorgang unzureichend war.

Insbesondere der Einsatz der Erfindung bei automatischen Melksystemen ist bevorzugt.

Bei automatischen Melksystemen ist eine räumliche Abteilung des Tierhinterteiles vorhanden, um das Tier örtlich besser zu fixieren. Dadurch wird der Raum kleiner, in dem ein Roboter oder dergleichen das Euter suchen muss. Weiterhin ist in automatischen Melksystemen oft eine Kamera vorhanden, mit der z.B. die Exkremente hinsichtlich des optischen Eindrucks untersucht werden können. Mit Hilfe einer Kamera oder eines Bildsensors können z.B. Farbe und Struktur der Exkremente untersucht werden.

Zwischen den Tierarten Schafen, Ziegen, Kamelen, Lamas, Pferden, Eseln, Büffeln und sonstigen Säugetieren, die sich zur Milchproduktion eignen, unterscheidet sich der Kot. Durch Auswertung zwei-, oder dreidimensionaler Bilder kann die Form und Farbe des Kots oder des Urins detektiert werden, um so tierindividuell und tierartenabhängig Rückschlüsse auf den Gesundheitsstatus zu erhalten. Bei der Auswertung kann auch die Art und die Menge des von dem jeweiligen Tier aufgenommenen Futters berücksichtigt werden, wenn diese Daten abrufbar sind. Dazu werden Tier- (Milchmenge, Melkhäufigkeit, Alter, Gewohnheiten etc.) -und Futterdaten (jeweilige Menge, Art und Zeitpunkt) vorzugsweise zentral gespeichert.

Mit einer Kamera oder dergleichen kann der Reinigungserfolg verifiziert werden. Ein eventuell vorhandener Greif- oder Roboterarm kann auch zur Probennahme verwendet oder zur anschließenden Reinigung einer Auffangeinrichtung oder des Kotbereiches eingesetzt werden.

Vorzugsweise wird erfindungsgemäß wenigstens eine Kenngröße und vorzugsweise wenigstens eine physikalische Kenngröße von den Exkrementen abgeleitet. Dabei kann z.B. die Temperatur, wenigstens eine Farbe, die Konsistenz, der Leitwert, die Viskosität, die Homogenität, der pH-Wert, oder die kapazitive Eigenschaft des Exkrements bestimmt werden. Bei inhomogenen Exkrementen können auch zwei oder mehr Kenngrößen für die jeweiligen Anteile bestimmt werden. Zusätzlich kann auch ein Maß für die Euter- und/oder Pansentemperatur bestimmt werden.

Bei manuellen oder halb oder auch vollautomatischen Melksystemen können eventuell vorhandene Sensoren auch zur Analyse eingesetzt werden.

Gemäß einer noch weiteren vorteilhaften Weiterbildung des Verfahrens wird vorgeschlagen, dass wenigstens eine Temperatur des Exkrements ermittelt wird. Insbesondere die bei der Defäkation aus dem Darm durch das Rektum ausgeschiedenen Exkremente weisen zunächst Körpertemperatur auf. Diese Temperatur wird mittels wenigstens eines Temperatursensors bestimmt, wobei die Wärmeabgabe bei der Temperaturbestimmung berücksichtigt werden kann.

Die Bestimmung der Körpertemperatur kann zusätzlich über die Ermittlung der Milchtemperatur erfolgen. Zusammen mit einer Milchmengenerfassung auf Euterviertelebene und/oder auf Tiergesamtgemelksebene, der Erfassung von Bewegungsaktivitäten und/oder Futteraufnahme sowie der Bestimmung von Milchinhaltsstoffen wie Harnstoffe, Ketonkörpern (Aceton, Acteoacetat und/oder β-Hydroxybutyrat), Fett, Eiweiß und/oder den Quotienten aus zwei oder mehreren Milchinhaltsstoffen (Fett/Eiweiß-Quotient) ist es möglich, die Gesundheit des Tieres noch genauer zu bestimmen.

Die Bestimmung der Temperatur des Tieres kann auch anhand der Ermittlung und Auswertung einer thermografischen Abbildung des Exkrements erfolgen.

Nach noch einer weiteren vorteilhaften Weiterbildung des Verfahrens wird vorgeschlagen, dass wenigstens eine elektrische Leitfähigkeit des Exkrements ermittelt wird. Eine Veränderung der elektrischen Leitfähigkeit kann zu einer Aussage über eine Beeinträchtigung des Gesundheitszustandes des Tieres führen.

Gemäß einer noch weiteren vorteilhaften Weiterbildung des Verfahrens wird vorgeschlagen, dass die Fäces (Kot) aufgefangen wird. Hierzu wird insbesondere vorgeschlagen, dass die Viskosität des Exkrements ermittelt wird.

Handelt es sich bei dem Kot um die Fäces einer Kuh, so weist diese eine unauffällige Konsistenz auf, wenn der Kot als mittelbreiig bezeichnet werden kann. Abweichungen von dieser Konsistenz (dünnbreiig bis dünnflüssig) können Hinweis auf Fütterungsfehler und/oder auf Erkrankungen des Tieres geben. Dazu führen rohfaserarmes Grünfutter (sog. Weidedurchfall), Rübenblattsilage und hohe Kraftfuttermengen. Dünnflüssiger Kot kann auch Ausdruck von Darmerkrankungen sein. In Abhängigkeit von der Ätiologie können diese Erkrankungen, die mit Durchfall einhergehen, auch Fieber, Milchleistungsrückgang bis zum Versiegen der Milch, Bewegungsunlust, Freßunlust und/oder Stoffwechselstörungen wie Ketosen zur Folge haben.

Nach einer noch weiteren vorteilhaften Ausbildung des Verfahrens wird vorgeschlagen, dass ein Stand für ein Tier vorgesehen ist, wobei eine Auffangeinheit in die Nähe einer kaudalen Körperregion des Tieres vorzugsweise zur Anlage gebracht wird. Insbesondere wird vorgeschlagen, dass die Auffangeinheit einer Bewegung des Tieres in dem Stand folgt. Hierbei wird sichergestellt, dass der Kot des Tieres auch in die Auffangeinheit gelangt, wenn sich das Tier in dem Stand befindet.

Nach noch weiteren erfinderischen Gedanken wird eine Einrichtung zur Bestimmung eines Gesundheitsstatus eines Tieres, insbesondere einer Kuh, vorgeschlagen, wobei die Einrichtung eine Auffangeinheit zum Auffangen wenigstens eines Teils eines Exkrements des Tieres aufweist. Es ist wenigstens eine Sensoreinrichtung zur Ermittlung wenigstens einer physikalischen Kenngröße des Exkrements vorgesehen. Die Sensoreinrichtung ist mit einer Auswerteeinrichtung zur Speicherung und Überprüfung verbunden. Bei der Überprüfung wird festgestellt, ob wenigstens die eine physikalische Kenngröße innerhalb eines vorgegebenen Toleranzfeldes liegt.

Die Auffangeinheit der Einrichtung weist eine Reinigungseinrichtung zur Reinigung wenigstens einer Sensoreinrichtung auf, so dass fehlerhafte Messungen durch verschmutzte Sensoren vermieden werden. Die aktuellen Exkremente werden nicht mit den Exkrementen eines vorhergehenden Tieres vermischt, da diese durch die Reinigungseinrichtung von der Sensoreinheit beseitigt worden sind.

Insbesondere wird vorgeschlagen, dass die Einrichtung Mittel aufweist, durch die der Reinigungserfolg überprüft und der Reinigungsvorgang gegebenenfalls wiederholt wird, wenn eine Überprüfung ergibt, dass der vorhergehende Reinigungsvorgang unzureichend war.

Die Reinigungseinrichtung weist vorzugsweise wenigstens eine Sprüheinheit auf, die ein Reinigungsmittel, bei dem es sich auch um Wasser handeln kann, zur Reinigung wenigstens einer Sensoreinrichtung benutzt.

Die Sensoreinrichtung weist vorzugsweise wenigstens einen Temperatursensor auf, durch den eine charakteristische Temperatur des Exkrements beim Ausscheiden ermittelt werden kann.

Nach noch einer weiteren vorteilhaften Ausbildung der Einrichtung wird vorgeschlagen, dass die Sensoreinrichtung wenigstens einen Sensor zur Messung einer elektrischen Leitfähigkeit des Exkrements aufweist.

Handelt es sich bei dem Exkrement um den Kot eines Tieres, so wird vorgeschlagen, dass die Sensoreinrichtung wenigstens eine Sensoreinheit zur Messung einer Viskosität des Exkrements weist. Die Viskosität des Kotes liefert eine relativ gesicherte Aussage über den Gesundheitszustand des Tieres. Aus diesem Wert können auch Schlussfolgerungen über Fressgewohnheiten und Trinkgewohnheiten des Tieres abgeleitet werden. Das ist sogar dann möglich, wenn z.B. die Abgabe von Futterart und Futtermenge nicht zentral gesteuert und gespeichert wird.

Gemäß einer noch weiteren vorteilhaften Ausgestaltung der Einrichtung wird vorgeschlagen, dass die Auffangeinheit einen Auffangbereich aufweist, der schräg nach oben gerichtet ist. Durch diese Ausgestaltung der Auffangeinheit wird die Bestimmung der Viskosität erleichtert. Des weiteren hat diese Ausgestaltung den Vorteil, die Reinigung der Auffangeinheit vereinfacht wird. Hierzu wird vorgeschlagen, dass die Sprüheinheit benachbart zur oberen Kante des Bereichs angeordnet ist. Es ist auch möglich, dass die Auffangeinheit beweglich angeordnet ist. Dann kann in z.B. einer ersten Position der Kot aufgefangen werden. Durch z.B. Hochklappen in eine zweite Position, in der das Auffangblech z.B. etwa senkrecht ausgerichtet ist, fällt ein Großteil des Kotes nach unten ab. Auch eine überhängende Stellung ist möglich. Auch ein Schieber kann zum Entfernen des Kotes vorgesehen sein.

Es wird vorgeschlagen, dass die Sensoreinrichtung wenigstens eine Lichtschranke umfasst. Vorzugsweise ist die wenigstens eine Lichtschranke derart angeordnet, dass der Lichtgang der wenigstens einen Lichtschranke im wesentlichen horizontal verläuft. Durch die wenigstens eine Lichtschranke wird die Möglichkeit geschaffen, den Kotabgang zu erfassen. Die Transmission des emittierten Lichts erlaubt es, zwischen Harn und Kot zu unterscheiden. Bei fehlender Transmission ist von einem erfolgreichen Kotabgang auf den Auffangbereich auszugehen.

Sind mehrere Lichtschranken (z.B. eine Zeilenkamera) vorgesehen, so können diese zur Bestimmung der Viskosität des Kotes herangezogen werden. Da normal geformter Kot eine mittel- bis festbreiige Konsistenz aufweist, ist der auf dem schräg angeordneten Auffangblech aufgefangene Kot geschichtet. Bei dünnbreiigen bzw. dünnflüssigen Kotabgängen wird hingegen keine Schichtung erreicht. Die Schichthöhe lässt sich durch die Lichtschranken erfassen. Beim Fehlen der Transmission des emittierten Lichts in Verbindung mit einer ermittelten, zu niedrigen Schichthöhe kann davon ausgegangen werden, dass ein Durchfall vorliegt. Fehlende Transmission des emittierten Lichtes bei ausreichender Schichthöhe weisen auf einen Normalbefund hin. Deutlich gemessene Transmission von Licht in Verbindung mit einer fehlenden Schichthöhe weisen auf Harnabgänge hin. Gleichzeitige Abgänge von Kot und Harn sind aus verhaltensphysiologischen und funktionellen Gründen nicht zu erwarten, wohl aber die unmittelbar hintereinander erfolgende Entleerung von Harnblase und Darm.

Zur Bestimmung der Viskosität bzw. der Kotkonsistenz wird gemäß einer noch weiteren vorteilhaften Ausgestaltung der Einrichtung vorgeschlagen, dass die Sensoreinrichtung wenigstens einen Drucksensor aufweist, der im Auffangbereich angeordnet wird. Wie bereits vorstehend ausgeführt, weist normal geformter Kot eine mittel- bis festbreiige Konsistenz auf. Der im Auffangbereich aufgefangene Kot ist geschichtet. Bei dünnbreiigen bzw. dünnflüssigen Kotabgängen wird hingegen keine Schichtung erreicht. Geschichteter bzw. geformter Kot rutscht den geneigten Auffangbereich zusammenhängend hinab. Ist im Auffangbereich wenigstens ein Drucksensor (oder Gewichtssensor) vorgesehen, so übt der Kot auf den Auffangbereich einen Druck aus. Beim Hinabrutschen des Kotes bildet sich die Druckhöhe aus. Aus der Höhe des Druckes und dessen zeitlicher Änderung kann auf die Konsistenz geschlossen werden.

Aus einer Erfassung einer zeitlichen Änderung ist es möglich, Rückschlüsse auf die Konsistenz zu erhalten. Dazu kann auch der bedeckte Flächenanteil bzw. dessen zeitliche Änderung ausgewertet werden, um so ein "Verlaufen" des Kotes auf einer Fläche messtechnisch zu erfassen.

In einer bevorzugten Weiterbildung werden biochemische Sensoren oder auch immonulogische Verfahren eingesetzt. Dazu kann auch eine Trennung des zu untersuchenden Exkrementes erfolgen. Diese kann beispielsweise über eine mechanische Trennung, ein Dekantieren, ein Separationsverfahren, ein Trennungsverfahren unter Ausnutzung von Zentrifugalkräften, magnetische und auch induktive Trennverfahren erfolgen.

Auch Polymerase Kettenreaktionen (Polymerase Chain Reactions, PCR) oder bakteriologische Verfahren können zur Bestimmung wenigstens eines Kennwertes verwendet werden. Ebenso ist ein Analyseverfahren über Bestimmun von Antikörpern oder eine Messung über ELISA (Enzym-verbundene immunoabsorbierende Proben (enzyme-linked immunoabsorbent assay)) ist möglich.

Bei der Analyse kann auch gezielt nach Salmonelloseerregern, Coli-Bakterien (auch E.coli 0157 oder EHEC), Mycobakterien, Brucellosebakterien, Rindertubukuloseerregern, Listeria oder Toxinbildnern gesucht werden. Zur Verstärkung können Reagenzien zugesetzt werden, die z.B. als Aktivator dienen. Dabei kann auch das emittierte Licht gemessen werden, das nach Zugabe eines Aktivators oder einer Reagenz ausgestrahlt wird und charakteristisch für bestimmte Stoffe ist.

Es kann auch die Reaktion eines Übertragungsmittels (transducer) gemessen werden, das charakteristisch für eine Reaktion von Inhaltsstoffen der Exkremente mit zugegebenen Reagenzien ist.

Die Messung kann auch die Analyse der optischen Eigenschaften umfassen. Insbesondere ist neben der Messung der Intensität des emittierten, transmitittierten oder reflektierten Lichtes auch die Bestimmung der Polarisationseigenschaften (lineare, elliptische oder z.B. zirkulare Polarisation) oder die Veränderung derselben durch die Messprobe möglich, da z.B. bestimmte Bakterien die Polarisationsrichtung des Lichts drehen.

Gemäß einer weiteren Ausgestaltung der Einrichtung wird vorgeschlagen, dass wenigstens der Auffangbereich mindestens im Bereich eines Temperatursensors aus einem wärmeisolierenden Werkstoff gebildet ist. Hierdurch wird sichergestellt, dass durch Wärmeverlust des Kotes an den Auffangbereich keine signifikante Änderung des Temperaturprofils bzw. der Temperatur des Kotes eintritt.

Nach einem noch weiteren erfinderischen Gedanken wird eine Vorrichtung zum automatischen Melken eines Tieres, insbesondere einer Kuh mit vorzugsweise wenigstens einem Stand und mit einer Melk- oder Fütter- oder Behandlungseinrichtung vorgeschlagen, wobei die Vorrichtung wenigstens eine Einrichtung nach einem der Ansprüche 9 bis 18 aufweist. Vorzugsweise weist die Vorrichtung wenigstens einen Melkstand auf, in dem die erfindungsgemäße Einrichtung angeordnet ist.

Nach einer weiteren vorteilhaften Ausbildung der Vorrichtung wird vorgeschlagen, dass die Einrichtung im Reinigungsstand angeordnet ist. Insbesondere wird vorgeschlagen, dass die Einrichtung so ausgebildet ist, dass diese wenigstens teilweise den Bewegungen des Tieres im Stand folgt.

In einer Weiterbildung einer oder mehrerer zuvor beschriebener Weiterbildungen wird vorzugsweise wenigstens eine elektrische Leitfähigkeit des Exkrements ermittelt. Bevorzugt ist in einer Weiterbildung, dass wenigstens eine Viskosität des Exkrements ermittelt wird.

Vorzugsweise wird wenigstens eine Fäzes- des Tieres aufgefangen. In verschiedenen Weiterbildungen ist es bevorzugt, dass das Reflektions- und/oder das Transmissionsvermögen des Exkrements bestimmt wird.

In einer Weiterbildung ist wenigstens eine Lichtschranke als Sensor vorgesehen, wobei vorzugsweise der Lichtgang wenigstens einer der wenigstens einen Lichtschranke im wesentlichen horizontal verläuft.

In einer Weiterbildung ist eine Lichtschranke vorgesehen, wobei der Lichtgang wenigstens einer der wenigstens einen Lichtschranke vorzugsweise im wesentlichen horizontal verläuft.

Weiterhin kann die Sensoreinrichtung wenigstens einen Drucksensor aufweisen, der vorzugsweise im Auffangbereich angeordnet ist.

Weitere Vorteile und Einzelheiten der Einrichtung werden anhand eines bevorzugten Ausführungsbeispiels welches in der Zeichnung dargestellt ist, erläutert, ohne dass der Gegenstand der Erfindung auf dieses Ausführungsbeispiel beschränkt wird.

Es zeigen:
Fig. 1 Schematisch eine Einrichtung in einer Vorderansicht,
Fig. 2 die Einrichtung in einer Seitenansicht und im Schnitt,
Fig. 3 die Einrichtung nach Fig. 1 in Verbindung mit einem Tier und
Fig. 4 die Einrichtung mit einem Tier nach Fig. 3 im ausgelenkten Zustand.

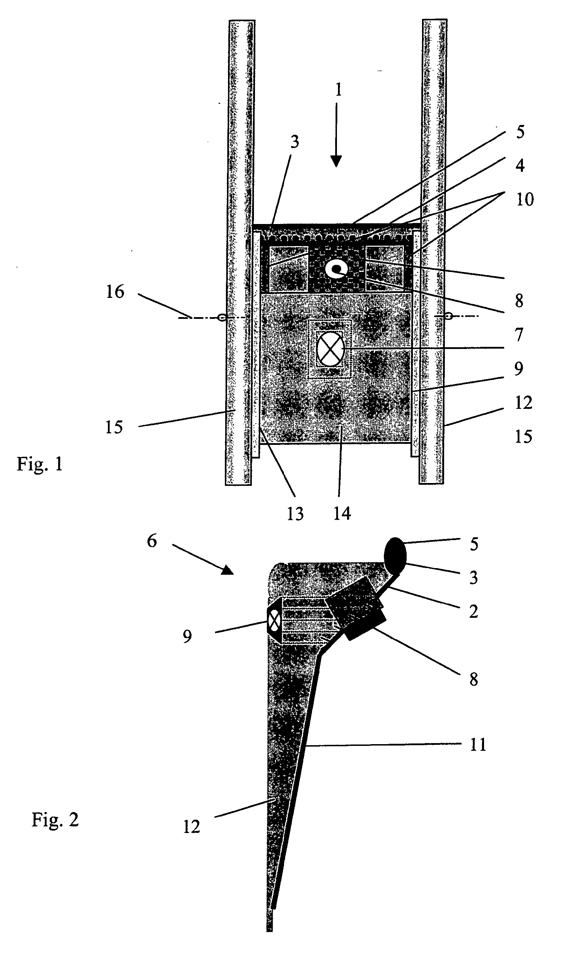

Fig. 1 und 2 zeigen eine Auffangeinheit 1 einer Einrichtung zur Bestimmung eines Gesundheitsstatus eines Tieres, insbesondere einer Kuh. Die Auffangeinheit 1 dient zum Auffangen wenigstens eines Teils eines Exkrements eines Tieres.

Die Auffangeinheit 1 weist einen Auffangbereich 2 auf, der schräg nach oben gerichtet ist, wie dies in der Fig. 2 dargestellt ist. Die Neigung des Auffangbereichs 2 ist derart gewählt, dass die Möglichkeit besteht, dass ein Exkrement, wenn dieses eine normale Konsistenz hat, über ein Teil des Auffangbereichs 2 hinunterrutschen kann, ohne dass das Exkrement den Auffangbereich 2 vollständig verlässt.

Die Auffangeinheit 1 weist eine Reinigungseinrichtung 3 zur Reinigung wenigstens des Auffangbereichs 2 auf. In dem dargestellten Ausführungsbeispiel ist die Reinigungseinrichtung 3 rohrförmig ausgebildet. Die Reinigungseinrichtung 3 ist benachbart zur oberen Kante des Auffangbereichs 2 angeordnet. Nicht dargestellt sind in der Fig. 1 bzw. 2 Versorgungsleitungen und gegebenenfalls eine Steuerung zur Durchführung eines Reinigungsvorgangs.

Die Reinigungseinrichtung 3 weist Öffnungen 4 auf, die vorzugsweise düsenförmig ausgebildet sind. Die Öffnungen 4 sind derart angeordnet, dass ein durch die Öffnungen 4 heraustretender Strahl eines Reinigungsmittels zur Reinigung wenigstens des Auffangbereichs 2 führt. Wenigstens ein Teil der Düsen ist vorzugsweise so ausgebildet, dass das Reinigungsmittel in Form eines Sprays heraustritt. Die rohrförmig ausgebildete Reinigungseinrichtung 3 weist eine Polsterung 5 auf, die zur Anlage an kaudale Körperpartien eines Tieres gelangt. Es ist nicht zwingend, dass eine Polsterung 5 vorgesehen ist. Die rohrförmig ausgebildete Reinigungseinrichtung 3 kann beispielsweise aus einem Kunststoff bestehen, so dass eine Verletzung des Tieres vermieden wird.

Aus der Fig. 2 ist insbesondere ersichtlich, dass die Auffangeinheit 1 eine Sensoreinrichtung 6 aufweist. Die Sensoreinrichtung 6 dient zur Ermittlung wenigstens einer physikalischen Kenngröße des Exkrements. Die Sensoreinrichtung 6 wird mit einer Auswerteeinrichtung (nicht dargestellt) verbunden. Die Auswerteeinrichtung ist zur Speicherung und Überprüfung ob wenigstens die eine physikalische Kenngröße innerhalb eines vorgegebenen Toleranzfeldes liegt, vorgesehen. Durch die Speicherung der physikalischen Kenngrößen und einer Zuordnung der Kenngrößen zu einem bestimmten Tier kann die zeitliche Veränderung der physikalischen Kenngrößen verifiziert werden. In Abhängigkeit vom Grad der Veränderung kann gegebenenfalls auch im Vorstadium eine pathologische Veränderung des Tieres festgestellt werden.

Die Sensoreinrichtung 6 weist einen Temperatursensor 7 auf, der im Auffangbereich 2 angeordnet ist. Um zu vermeiden, dass Wärmeverluste entstehen, die zu einer verfälschten Temperatur des Kotes führen, ist der Auffangbereich 2 vorzugsweise aus einem wärmeisolierenden Werkstoff gebildet.

In dem dargestellten Ausführungsbeispiel ist der Temperatursensor 7 innerhalb eines Sensorfeldes 8 angeordnet. Das Sensorfeld 8 besteht vorzugsweise aus Fotozellen. Eine Lichtquelle 9 emittiert Licht, welches auf die Fotozellen des Sensorfeldes 8 auftritt. Durch das Sensorfeld 8 wird verifiziert, ob sich überhaupt Kot auf dem Auffangbereich 2 befindet. Andere Exkremente wie z.B. Harn sind lichtdurchlässig, so dass das Sensorfeld ein Signal erzeugt, wenn Kot auf dem Auffangbereich 2 vorhanden ist. Durch dieses Signal wird eine Auswerteinrichtung aktiviert.

An den Längsrändern des Auffangbereichs sind Lichtschranken 10 vorgesehen. Die Lichtschranken sind durch entsprechende Lichtsensoren gebildet. Die Lichtschranken weisen mehrere Lichtsensoren auf, die übereinander und nebeneinander in einer senkrecht zum Auffangbereich 2 stehenden Ebene angeordnet sind. Diese Lichtschranken bzw. Lichtsensoren dienen zur Bestimmung der Schichthöhe von Kot. Ist der Kot dickbreiig, so ist die Schichtung ausgeprägt. Bei Durchfall ist keine oder nur im geringen Maße eine Schichtung ausgeprägt. Aus der Höhe sowie aus dem zeitlichen Verlauf der Ausbildung der Schichthöhe kann die Viskosität des Kotes rückgeschlossen werden.

An den Auffangbereich 2 schließt sich ein Abführbereich 11 an. Er verläuft steiler als der Auffangbereich 2, so dass der Kot bzw. Verunreinigungen, die sich auf dem Auffangbereich 2 befinden leichter abgeführt werden können. Nicht dargestellt ist eine Leitung durch die die Exkremente und Reinigungsmittel abgeführt werden.

Der Auffangbereich 2 und der Abführbereich 11 können einteilig ausgebildet sein.

Die Auffangeinheit 1 weist zwei parallel zueinander verlaufende und zueinander beabstandete Wände 12, 13 zwischen denen der Auffangbereich 2 und der Abführbereich 11 angeordnet ist. Des weiteren ist eine Abdeckung 14 vorgesehen. In dem dargestellten Ausführungsbeispiel ist die Auffangeinheit 1 zwischen zwei parallel zueinander angeordneten im wesentlichen vertikal verlaufenden Trägern 15 angeordnet. Die Seitenwände 12, 13 sind mit den Trägern 15 verbunden. Die Verbindung ist derart ausgebildet, dass die Auffangeinheit 1 um eine Schwenkachse 16 verschwenkbar ist.

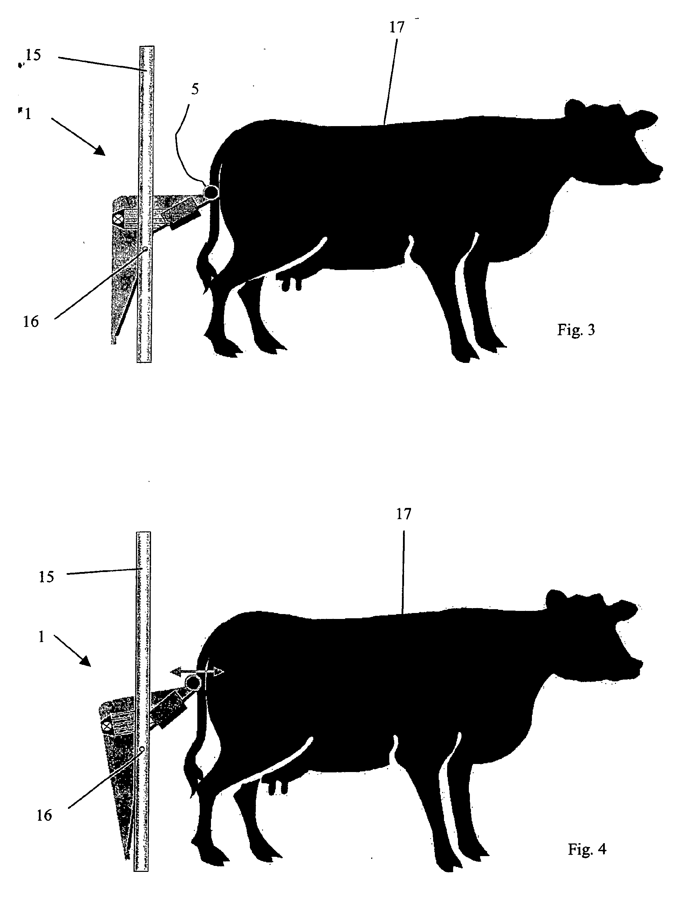

Fig. 3 und 4 zeigen das Zusammenspiel eines Tieres 17 mit der Auffangeinheit 1. Das Tier 17 gelangt mit seinen kaudalen Körperpartien an die Polsterung 5. Bewegt sich das Tier in Richtung der Träger 15, so schwenkt die Auffangeinheit 1 aus. Dies erfolgt somit mit der rückwärtigen Bewegung des Tieres 17. Bewegt sich das Tier 17, bei dem es sich hier im Ausführungsbeispiel um eine Kuh handelt, vorwärts, so schwenkt die Auffangeinheit 1 nach vorn. Die Erfindung kann auch bei anderen milchabgebenden Säugetieren eingesetzt werden. Das Anschmiegen der Auffangeinheit 1 an die kaudalen Körperpartien des Tieres 17 sollte mit einem Anpressdruck erfolgen, der es dem Tier erlaubt, den Schwanz zur Defäkation über die Auffangeinheit 1 zu heben. Dazu kann die Auffangeinheit 1 auch direkt unterhalb des Schwanzes positioniert werden. Die Auffangeinheit sollte unterhalb der Kotaustrittsstelle angeordnet werden.

Die Einrichtung zur Bestimmung eines Gesundheitsstatus eines Tieres insbesondere geeignet für eine Vorrichtung zum automatischen Melken eines Tieres. Die Einrichtung kann in einen Melkstand oder in einen Reinigungsstand integriert werden. Wo nicht sichergestellt werden kann, dass es während eines Melkvorgangs oder während eines Reinigungsvorgangs zu einer Defäkation kommt, kann davon ausgegangen werden, dass die Wahrscheinlichkeit einer Defäkation relativ hoch ist, wenn das Tier Durchfall hat.

### Bezugszeichenliste

- 1: Auffangeinheit
- 2: Auffangbereich
- 3: Reinigungseinrichtung
- 4: Öffnung
- 5: Polsterung
- 6: Sensoreinrichtung
- 7: Temperatursensor
- 8: Sensorfeld
- 9: Lichtquelle
- 10: Lichtschranke
- 11: Abführbereich
- 12: Seitenwand
- 13: Seitenwand
- 14: Abdeckung
- 15: Träger
- 16: Schwenkachse
- 17: Tier

## Patentansprüche

1. Verfahren zur Bestimmung eines Gesundheitsstatus eines Tieres (17),
insbesondere einer Kuh, **gekennzeichnet durch**
Auffangen wenigstens eines Teils eines Exkrements des Tieres (17),
Ermittlung und Speicherung wenigstens einer physikalischen Kenngröße des Exkrements,
Überprüfung ob wenigstens die eine physikalische Kenngröße innerhalb eines vorgegebenen Toleranzfeldes liegt.

2. Verfahren nach Anspruch 1, bei dem vor dem Auffangen des Exkrements mittels einer Auffangeinheit (1) diese gereinigt wird, wobei die Reinigung vorzugsweise mit einem flüssigen Mittel erfolgen kann.

3. Verfahren nach Anspruch 2, bei dem der Reinigungsvorgang wiederholt wird, wenn eine Überprüfung ergibt, dass der vorhergehende Reinigungsvorgang unzureichend war.

4. Verfahren nach mindestens einem der vorhergehenden Ansprüche, bei dem wenigstens eine physikalische Kenngröße ermittelt wird, welche aus einer Gruppe von physikalischen Kenngrößen entnommen ist, wobei diese Gruppe der physikalischen Kenngrößen eine Temperatur, eine elektrische Leitfähigkeit, eine Viskosität, ein Reflektionsvermögen bei wenigstens einer Wellenlänge, ein Transmissionsvermögen bei wenigstens einer Wellenlänge, eine Farbe, einen pH-Wert, eine Kapazität, eine Induktivität des Exkrements und dergleichen mehr umfasst.

5. Verfahren nach mindestens einem der vorhergehenden Ansprüche, bei dem eine thermographische Abbildung des Exkrements ermittelt wird.

6. Verfahren nach mindestens einem der vorhergehenden Ansprüche, bei dem wenigstens zwei physikalische Kenngrößen ermittelt werden.

7. Verfahren nach mindestens einem der vorhergehenden Ansprüche, wobei ein Stand für ein Tier (17) vorgesehen ist, bei dem eine Auffangeinheit (1) an eine kaudale Köperregion des Tieres (17) zur Anlage gebracht wird.

8. Verfahren nach mindestens einem der vorhergehenden Ansprüche, bei dem die Auffangeinheit (1) einer Bewegung des Tieres (17) in dem Stand folgt.

9. Einrichtung zur Bestimmung eines Gesundheitsstatus eines Tieres (17),
insbesondere einer Kuh, **gekennzeichnet durch**
eine Auffangeinheit (1) zum Auffangen wenigstens eines Teils eines Exkrements des Tieres (17),
wenigstens eine Sensoreinrichtung (6) zur Ermittlung wenigstens einer physikalischen Kenngröße des Exkrements und
eine Auswerteeinrichtung zur Speicherung und Überprüfung ob wenigstens die eine physikalische Kenngröße innerhalb eines vorgegebenen Toleranzfeldes liegt.

10. Einrichtung nach Anspruch 9, **dadurch gekennzeichnet, dass** wenigstens eine Reinigungseinrichtung (3) zur Reinigung wenigstens eines Bereichs der Auffangeinheit (1) vorgesehen ist.

11. Einrichtung nach Anspruch 9 oder 10, **dadurch gekennzeichnet, dass** Mittel vorgesehen sind, durch die der Reinigungserfolg überprüft und gegebenenfalls wiederholt wird, wenn eine Überprüfung ergibt, dass der vorhergehende Reinigungsvorgang unzureichend war.

12. Einrichtung nach Anspruch 10 oder 11, **dadurch gekennzeichnet, dass** die Reinigungseinrichtung (3) eine Sprüheinheit aufweist.

13. Einrichtung nach einem der Ansprüche 9 bis 12, **dadurch gekennzeichnet, dass** die Sensoreinrichtung (6) wenigsten einen Sensor umfasst, welcher einer Gruppe von Sensoren entnommen ist, welche einen Temperatursensor (7), einen Sensor zur Messung einer elektrischen Leitfähigkeit des Exkrements, eine Sensoreinheit zur Ermittlung einer Viskosität des Exkrements, eine Sensoreinheit zur Ermittlung eines pH-Wertes des Exkrements, einen Drucksensor, eine Sensoreinheit zur Bestimmung des transmittierten Lichtes, eine Sensoreinheit zur Bestimmung des reflektierten Lichtes, eine Kapazitätssensor, einen Induktivitätssensor, einen Bildsensor und dergleichen mehr umfasst.

14. Einrichtung nach einem der Ansprüche 9 bis 13, bei dem wenigstens zwei physikalische Kenngrößen ermittelt werden.

15. Einrichtung nach einem der Ansprüche 9 bis 14, **dadurch gekennzeichnet, dass** die Auffangeinheit (1) einen Auffangbereich (2) aufweist, der schräg nach oben gerichtet ist, wobei die Reinigungseinrichtung (3) vorzugsweise im wesentlichen benachbart zu der oberen Kante des Auffangbereichs (2) angeordnet ist.

16. Einrichtung nach einem der Ansprüche 9 bis 15, **dadurch gekennzeichnet, dass** der Auffangbereich (2) wenigstens eine dieser wenigstens einen Sensoreinrichtung (6) aufweist.

17. Einrichtung nach einem der Ansprüche 9 bis 16, **dadurch gekennzeichnet, dass** die Sensoreinrichtung (6) wenigstens eine Lichtschranke (10) umfasst, wobei vorzugsweise der Lichtgang wenigstens einer der wenigstens einen Lichtschranke (10) im wesentlichen horizontal verläuft.

18. Einrichtung nach einem der Ansprüche 9 bis 17, **dadurch gekennzeichnet, dass** wenigstens der Auffangbereich (2) mindestens im Bereich eines Temperatursensors (7) aus einem wärmeisolierenden Werkstoff gebildet ist.

19. Vorrichtung zum Versorgen eines Tieres, insbesondere einer Kuh, welche wenigstens eine Melkeinrichtung und/oder wenigsten eine Füttereinrichtung und/oder wenigstens eine Reinigungseinrichtung umfasst und wobei weiterhin wenigstens eine Einrichtung zur Bestimmung eines Gesundheitsstatus eines Tieres nach einem der Ansprüche 9 bis 18 vorgesehen ist.
